# EUROPEAN PATENT APPLICATION

(11) **EP 2 322 122 A1**
(43) Date of publication of application: **18.05.2011**
(21) Application number: 10191436.4
(22) Date of filing: 16.11.2010
(51) Int. Cl.: A61F 2/84

(54) **System for positioning a stent**

(30) Priority: 16.11.2009 CN 200920256629 U
(71) Applicant: Micro-Tech Europe GmbH, 40472 Düsseldorf (DE)
(72) Inventor: Changwang, Pan, Nanjing (CN); Yuqian, Li, Nanjing (CN); Derong, Leng, Xiaguan District Nanjing (CN)
(74) Representative: Albrecht, Ralf

(57) **Abstract**

The present invention relates to a sort of Accurate Proximal Positioning Stent Delivery System, which is comprised of an olive tip (1), an inner tube (2), a radiopaque marker (4), a middle tube (8), an outer tube (5), a front handle (6), a safety lock (7), a positioning block (9), a back handle (10), a stainless steel tube (11), a loop assembly (12), and a handle with a flushing port (13). The olive tip is fixed at a front-end of the inner tube; the outer tube is joined with the front handle; the middle tube is joined with the back handle; the back-end of the inner tube is connected with the stainless steel tube and the handle with flushing port; and the loop assembly binds and fixes the stent on the exterior of the inner tube to position accurately. The delivery system of the present invention can position accurately and adjust the stent during the procedure of the stent's deployment, improve the success of surgical operation, reduce the difficulties and risks of surgery, and can be operated in a safe, convenient, and reliable way.

## Description

### TECHNICAL FIELD OF THE INVENTION

The present invention relates to a sort of medical device, to be specific, a sort of Accurate Proximal Positioning Stent Delivery System.

### BACKGROUND OF THE INVENTION

At present, usually it is inclined to implant the stent in the treatment of stenosis of esophagus, trachea, bile duct and prostate so forth produced by malignant neoplasm and cancer so on. Delivery system is the necessity for stent implantation. Presently, the conventional delivery system has no positioning property, and is unable to adjust and confirm the deployment position during the procedure of implantation for many times, to some extent, which reduces the success of the surgery, and increases the surgical risks as well.

### SUMMARY OF THE INVENTION

The present invention is directed to solve the above-said problem by providing a sort of Accurate Proximal Positioning Stent Delivery System with convenient operation.

The technical solution of the present invention is as follows:
The Accurate Proximal Positioning Stent Delivery System consists of the olive tip (1), the inner tube (2), the radiopaque marker (4), the middle tube (8), the outer tube (5), the front handle (6), the safety lock (7), the positioning block (9), the back handle (10), the stainless steel tube (11), the loop assembly (12), and the handle with flushing port (13) so on. The olive tip (1) is fixed in the front-end of inner tube (2); the outer tube (5) is joined with the front handle (6); the middle tube (8) is joined with the back handle (10); the back-end of the inner (2) is connected with stainless steel tube (11) and handle with flushing port (13); and
the loop assembly (12) binds and fixes the stent (3) on the exterior of the inner tube (2).

Compared with the homogeneous stent, the present invention has the features as follows:
a. The delivery system of the present invention can position the stent for many times and adjust the stent deployment location before the stent has not been released completely.
b. The delivery system of the present invention is equipped with positioning block which can avoid the impossibility of retrieval when the stent is released completely during the procedure of operation, and provides the opportunity of re-adjusting when the location of stent is not accurate.
c. The delivery system of the present invention can be equipped with the safety lock which can effectively avoid the mis-manipulation during the procedure of operation.

### BRIEF DESCRIPTION OF DRAWINGS

Figure 1 is the structure sketch-map of the delivery system with one loop assembly of the present invention.
Figure 2 is the structure sketch-map of the delivery system with another loop assembly of the present invention.
Figure 3 is the structure sketch-map of one loop assembly of the present invention.
Figure 4 is the structure sketch-map of another loop assembly of the present invention.
Figure 5 Delivery system with one loop assembly before stent releasing
Figure 6 Releasing the stent out of the outer tube
Figure 7 Pull the stainless steel lasso (14) to loose the knot which fix the proximal end of the stent to release the stent
Figure 8 Stent be released fully
Figure 9 Delivery system with another loop assembly before stent releasing
Figure 10 Release the stent out of the outer tube
Figure 11 Pull the binding loop (17) to loose the knot which fix the proximal end of the stent to release the stent
Figure 12 Stent be released fully

### DETAILED DESCRIPTION OF THE INVENTION

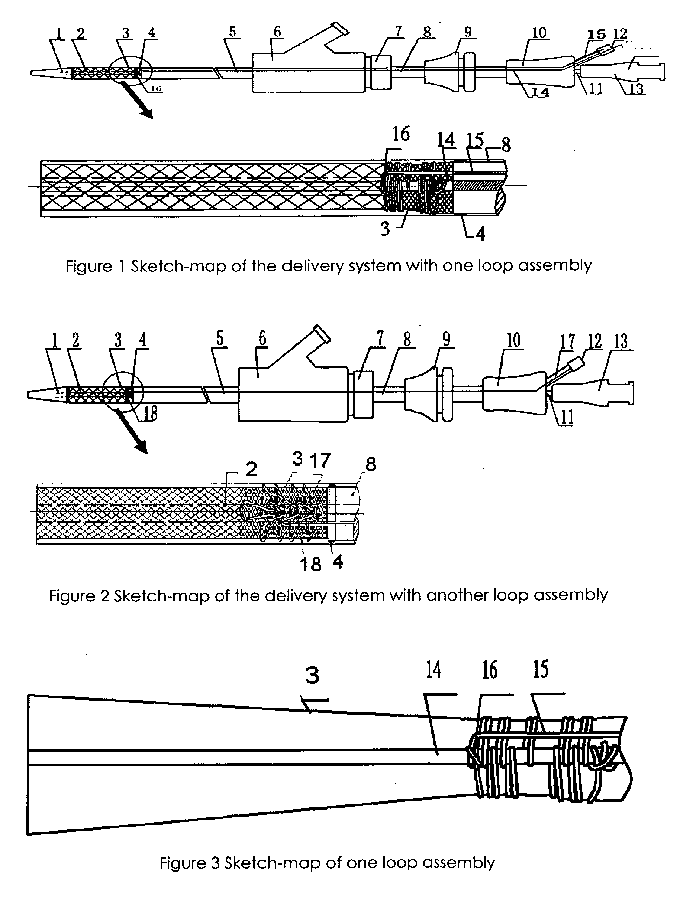

Describe one embodiment of the present invention based on the sketch-map annexed as follows. (Refer to Figure 1 & 3)

The Accurate Proximal Positioning Stent Delivery System (Refer to Figure 1) consists of olive tip (1), inner tube (2), radiopaque marker (4), middle tube (8), outer tube (5), front handle (6), safety lock (7), positioning block (9), back handle (10), stainless steel tube (11), loop assembly (12), and handle with flushing port (13) so on. The olive tip (1) is fixed in the front-end of inner tube (2); the outer tube (5) is joined with the front handle (6); the middle tube (8) is joined with the back handle (10); the back-end of the inner (2) is connected with stainless steel tube (11) and handle with flushing port (13); the stainless steel lasso (14) and the binding thread (15) bind and fix the proximal of the stent (3) on the exterior of the inner tube (2), and form into the slipknot (16) (Refer to Figure 3).

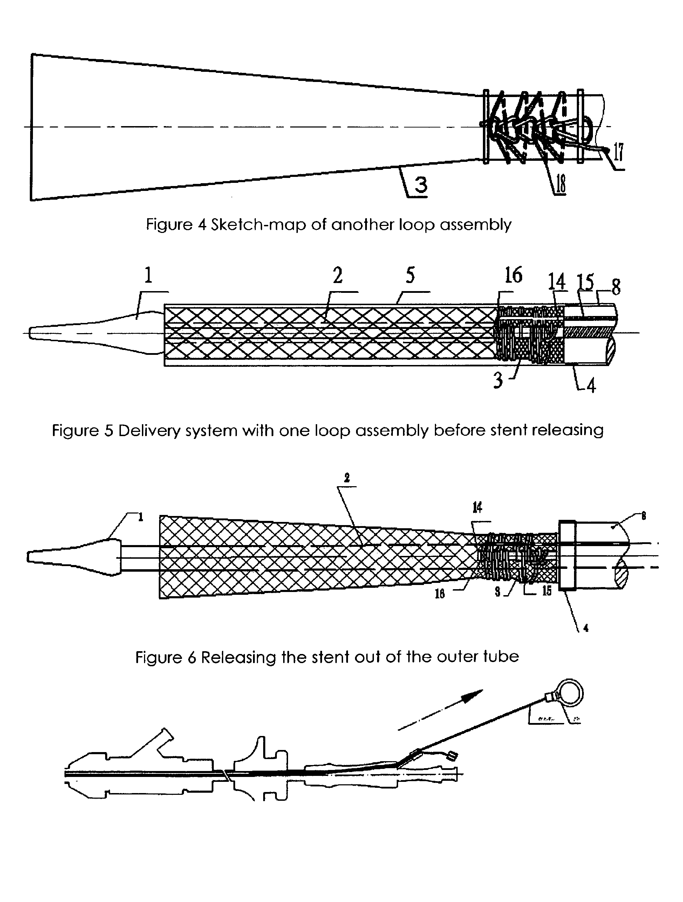

During the procedure of implanting, insert the delivery system of the present invention along with the guide wire to the pre-concerted position of the stent (3). Hold the back handle (10), withdraw the front handle (6) to the location of the positioning block (9). At this time, the proximal of the stent (3) is fixed by the loop assembly (12) with about 1 cm unreleased. If the deployed position is lower than the pre-concerted and the distance is short, pull the back handle (10), and take the stent (3) to the proper position. If the deployed position is lower than the pre-concerted and the distance is quite long, or the deployed position is higher than the pre-concerted, hold the back handle (10), push the front handle (6), compress the stent (3) into the outer tube (5) of the delivery system again, and re-adjust the position and implant it again. When confirm the proper position of the stent's releasing, loosen the positioning block (9), fix the back handle (10), withdraw the front handle (6), and allow the stent out of the outer tube (5) completely. Then fix the back handle (10), pull the stainless lasso (14), loosen the slipknot (16), and release the proximal of the stent (3). Pull the stainless steel lasso (14) and the binding thread (15) entirely to release the whole stent (3) completely, at last, take the delivery system out of the patient's body safely. (Refer to Figure 5 to 8)

The Accurate Proximal Positioning Stent Delivery System of the present invention can effectively position the stent, avoid the inaccurate implantation resulting from mis-manipulation or the length change of the stent itself in the procedure of operation, and improve the accuracy and success of the surgery.

Describe further another embodiment of the present invention based on sketch-map annexed as follows. (Refer to Figure 2 & 4)

The Accurate Proximal Positioning Stent Delivery System (Refer to Figure 2) consists of olive tip (1), inner tube (2), radiopaque marker (4), middle tube (8), outer tube (5), front handle (6), safety lock (7), positioning block (9), back handle (10), stainless steel tube (11), loop assembly (12), and handle with flushing port (13) so on. The olive tip (1) is fixed in the front-end of inner tube (2); the outer tube (5) is joined with the front handle (6); the middle tube (8) is joined with the back handle (10); the back-end of the inner (2) is connected with stainless steel tube (11) and handle with flushing port (13); the binding loop (17) binds and fixes the proximal of the stent (3) on the exterior of the inner tube (2), and form into the slipknot (18) (Refer to Figure 4).

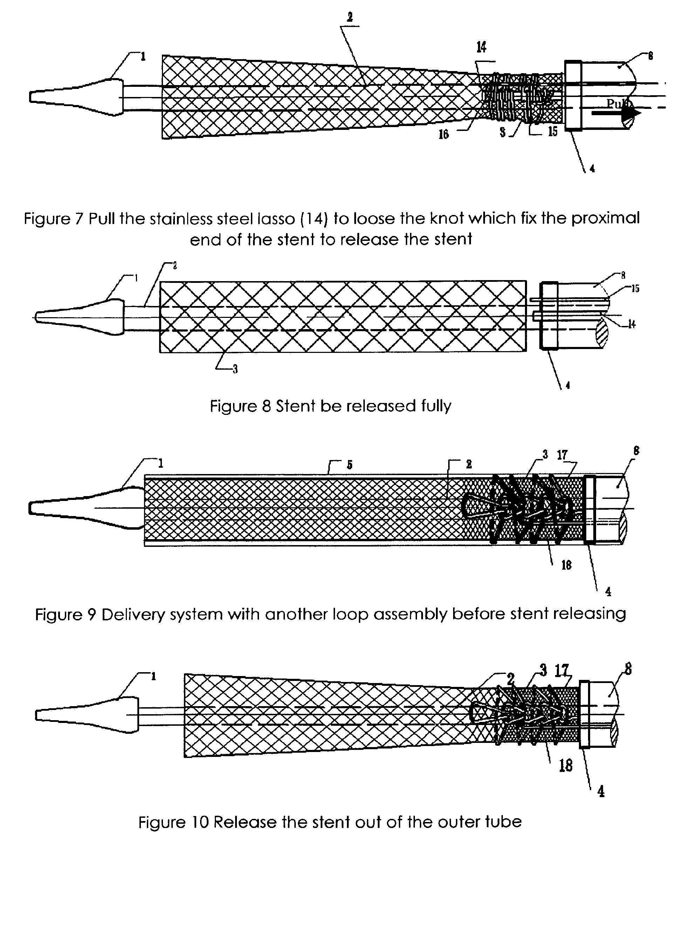

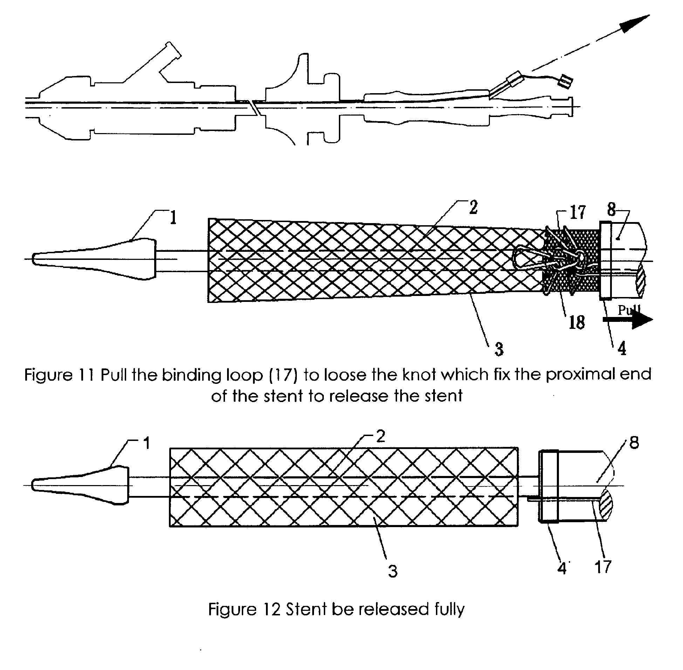

During the procedure of implanting, insert the delivery system of the present invention along with the guide wire to the pre-concerted position of the stent (3). Hold the back handle (10), withdraw the front handle (6) to the location of the positioning block (9). At this time, the proximal of the stent (3) is fixed by the loop assembly (12) with about 1 cm unreleased. If the deployed position is lower than the pre-concerted and the distance is short, pull the back handle (10), and take the stent (3) to the proper position. If the deployed position is lower than the pre-concerted and the distance is quite long, or the deployed position is higher than the pre-concerted, hold the back handle (10), push the front handle (6), compress the stent (3) into the outer tube (5) of the delivery system again, and re-adjust the position and implant it again. When confirm the proper position of the stent's releasing, loosen the positioning block (9), fix the back handle (10), withdraw the front handle (6), and allow the stent out of the outer tube (5) completely. Then fix the back handle (10), pull the binding loop (17), loosen the slipknot (18), and release the proximal of the stent (3). Pull the binding loop (17) entirely to release the whole stent (3) completely, at last, take the delivery system out of the patient's body safely. (Refer to Figure 9 to 12)

The Accurate Proximal Positioning Stent Delivery System of the present invention can effectively position the stent, avoid the inaccurate implantation resulting from mis-manipulation or the length change of the stent itself in the procedure of operation, and improve the accuracy and success of the surgery.

The above disclosure is intended to be illustrative and not exhaustive. These examples and description will suggest may variations and alternatives to one of ordinary skill in the art. All these variations and alternatives are intended to be included within the scope of the attached claims. Those familiar with the may recognize other equivalents to the specific embodiment disclosed herein which equivalents are also intended to be encompassed by the claims attached here to.

## Claims

1. The characteristic of the Accurate Proximal Positioning Stent Delivery System is that it consists of a three-catheter system comprised of the outer tube (5), the middle tube (8) and the inner tube (2), the olive tip (1) fixed in the flange of the inner tube (2), the stent (3), the loop assembly (12) which binds and fixes one end of the stent (3) on the exterior of the inner tube (2), the front handle (6) and the back handle (10) so on.

2. According to claim 1, wherein the characteristic of the said Accurate Proximal Positioning Stent Delivery System is that it can attach the radiopaque marker (4) on the inner tube (2), and can be equipped with the safety lock (7), the positioning block (9), the pushing aids tube (11), and the handle with flushing port (13).

3. According to claim 1, wherein the characteristic is that the said loop assembly (12) can fix the proximal end of the stent (3) and the distal end of the stent (3) as well, wherein accordingly it has two binding embodiments of fixing the stent (3) in the proximal end and the distal end independently.

4. According to claim 1, wherein the characteristic is that the said loop assembly (12) can be the slipknot (16) formed by the stainless steel lasso (14) and the binding loop (15) to bind and position the stent (3).

5. According to claim 1, wherein the characteristic is that the said loop assembly (12) can be the slipknot (18) formed by the binding loop (17) to bind and position the stent (3).
